Europäisches Patentamt

European Patent Office

Office européen des brevets

(19)

(11) Veröffentlichungsnummer : **0 174 625 B1**

(12) **EUROPÄISCHE PATENTSCHRIFT**

(45) Veröffentlichungstag der Patentschrift :
23.11.89

(21) Anmeldenummer : 85111342.3

(22) Anmeldetag : 07.09.85

(51) Int. Cl.⁴ : **C 07 D307/89// C07D307/60, F28C3/02**

(54) **Verfahren zur Erhöhung der Temperatur des Abgases aus der Abgaswaschanlage bei der Herstellung von Phthalsäureanhydrid.**

(30) Priorität : 12.09.84 DE 3433402

(43) Veröffentlichungstag der Anmeldung :
19.03.86 Patentblatt 86/12

(45) Bekanntmachung des Hinweises auf die Patenterteilung : 23.11.89 Patentblatt 89/47

(84) Benannte Vertragsstaaten :
AT BE DE FR GB IT

(56) Entgegenhaltungen :
DE–A– 2 948 163
ENERGIE, 33. Jahrgang, Heft 12, Dezember 1981, Techn. Verlag Resch KG, Gräfelfing: "Wieder aufheizen wie?", Seiten 404-407
ULLMANNS ENCYKLOPÄDIE DER TECHNISCHEN CHEMIE, 3. Auflage, 1. Band, 1951, Verlag Urban & Schwarzenberg, München, Seiten 256-258
Die Akte enthält technische Angaben, die nach dem Eingang der Anmeldung eingereicht wurden und die nicht in dieser Patentschrift enthalten sind.

(73) Patentinhaber : BASF Aktiengesellschaft
Carl-Bosch-Strasse 38
D-6700 Ludwigshafen (DE)

(72) Erfinder : Block, Ulrich, Dr.
Ungsteiner Strasse 14
D-6700 Ludwigshafen (DE)

EP 0 174 625 B1

## Beschreibung

Die Erfindung betrifft ein Verfahren zur Erhöhung der Temperatur des Abgases aus der Abgaswaschanlage (im folgenden Abgas genannt) bei der Herstellung von Phthalsäureanhydrid (PSA) durch Katalytische Luftoxidation von Naphthalin oder o-Xylol.

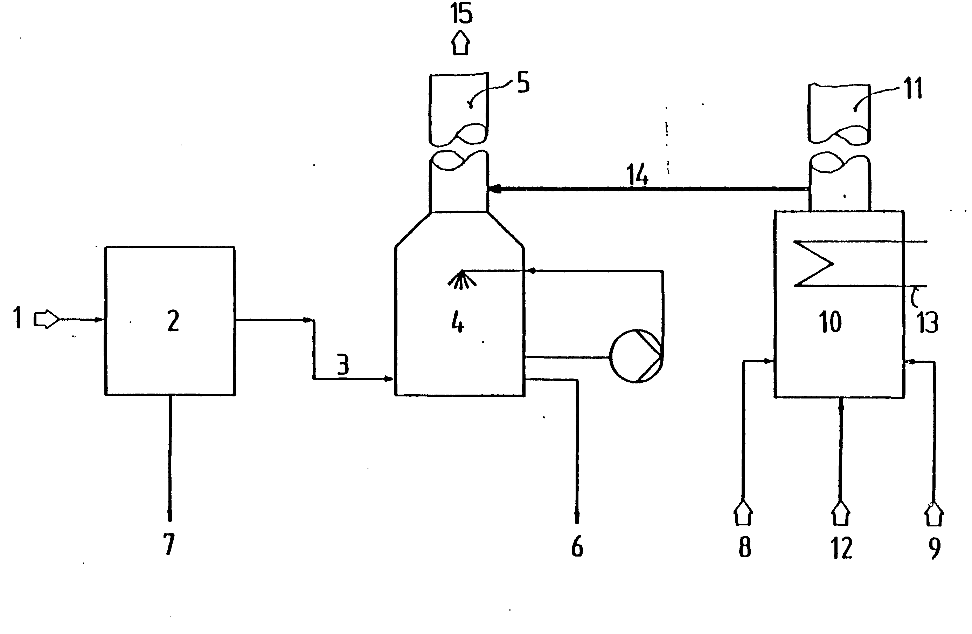

Bei dem verfahren zur Herstellung von PSA durch katalytische Luftoxidation von Naphthalin oder o-Xylol — wie in dem Nachschlagewerk « Ullmanns Encyklopädie der technischen Chemie », 4. Auflage, Band 18, Abb. 4, S. 527 beschrieben — wird nach der Wäsche mit Waschlösung ein Abgas von 30 bis 50 °C erhalten, das durch einen Kamin in die Atmosphäre austritt. Die niedrige Temperatur des Abgases bedingt einerseits wegen der geringen thermischen Überhöhung eine große Kaminhöhe und andererseits kommt es bei Abkühlung des Abgases im Kamin zur Taupunktsunterschreitung, was Tropfenauswurf aus dem Kamin zur Folge hat.

Bis heute wurden diese Nachteile bei den entsprechenden Anlagen nicht beseitigt. Eine Lösung ist im Stand der Technik nicht aufgezeigt.

Der Erfindung liegt daher die Aufgabe zugrunde, die Nachteile zu vermeiden, die durch die niedrige Temperatur des Abgases beim Austritt in die Atmosphäre bedingt sind.

Zur Vermeidung dieser Nachteile könnte beispielsweise zur Erhöhung der Temperatur des Abgases ein Teil des heißen, ungereinigten Abgases vor der Abgaswaschanlage abgezweigt und mit dem kälteren, gereinigten Abgas nach der Abgaswaschanlage wieder zusammengeführt werden. Dies hat jedoch zur Folge, daß teilweise ungereinigtes Abgas — was umweltbeeinträchtigend ist — in die Atmosphäre gelangt. Auch das Aufwärmen des Abgasstromes mittels dampfbeheizter Abgasvorwärmer oder öl- oder erdgasbefeuerter Heißgaserzeuger ist — aufgrund hohen Energieverbrauchs — unvorteilhaft (s. Zeitschrift Energie, JG. 33, Nr. 12, Dez. 1981, Seiten 404 bis 407).

Die gestellte Aufgabe wird erfindungsgemäß dadurch gelöst, daß dem Abgas vor dem Austritt in die Atmosphäre Verbrennungsabgas aus dem Verbrennungsofen für Rückstände aus den PSA- und Maleinsäureanhydrid-Destillationseinheiten zugemischt wird.

Weitere Merkmale des erfindungsgemäßen Verfahrens sind Gegenstand der Unteransprüche.

Das Abgas und das Verbrennungsabgas aus dem Verbrennungsofen — in dem die jeweils leichtsiedenden Verunreinigungen und die schwersiedenden Rückstände aus den PSA- und Maleinsäureanhydrid-Destillationseinheiten verbrannt werden — fallen bei dem Verfahren zur Herstellung von PSA im Mengenverhältnis 20 : 1 bis 5 : 1 an. Dieses Mengenverhältnis ist abhängig von der speziellen Ausgetaltung des Verfahrens und ist ohne Einfluß auf die Wirksamkeit der vorliegenden Erfindung. Die Temperatur des Verbrennungsabgases beträgt 500 bis 800 °C und im Fall einer Wärmenutzung beträgt die Temperatur des Verbrennungsabgases 500 bis 200 °C. Durch die Zumischung des Verbrennungsabgases in den Abgasstrom erhält man in Abhängigkeit des Mengenverhältnisses der beiden Abgase zueinander und in Abhängigkeit der Temperatur des Verbrennungsabgases eine Erhöhung der Temperatur des Abgases um 10 bis 100 °C. Diese Temperaturerhöhung ist ausreichend um das Abgas von dem bezüglich Wasserdampf gesättigten Zustand in den überhitzten Zustand überzuführen, wodurch Tropfenauswurf aus dem Kamin verhindert wird. Des weiteren führt eine größere thermische Überhöhung des Abgases zu einer kleineren Bauhöhe des Kamins. Deshalb wird man bestrebt sein, das gesamte Verbrennungsabgas den Abgas zuzumischen, was als weiteren Vorteil der vorliegenden Erfindung einen Wegfall des Kamins des Verbrennungsofens zur Folge hätte.

Ein Ausführungsbeispiel des erfindungsgemäßen Verfahrens ist in der Zeichnung dargestellt und wird im folgenden näher beschrieben. Die Zeichnung zeigt ein vereinfachtes Verfahrensschema.

Gemäß dem Verfahrensschema wird das aus dem Reaktor austretende und anschließend auf ca. 180 °C gekühlte Reaktionsgas 1 in den Abscheider 2 geleitet, in dem ungereinigtes PSA fest abgeschieden wird. Der im Abscheider auf ca. 50 °C abgekühlte Gasstrom 3 wird zur Abgaswaschanlage 4 geführt, wo der Gasstrom durch eine Wasserwäsche gereinigt wird und über den Kamin 5 in die Umgebung abgegeben wird, während die bei der Wäsche anfallende, wäßrige Maleinsäure aus der Abgaswaschanlage als Strom 6 abgezogen wird, aus dem in bekannter Weise reines MSA gewonnen wird. Das ungereinigte PSA wird im Abscheider abgeschmolzen und als Strom 7 abgezogen, aus dem in bekannter Weise reines PSA gewonnen wird. Die aus den Aufarbeitungsapparaturen für reines PSA und reines MSA anfallende leichtsiedende Verunreinigungen 8 und schwersiedende Rückstände 9 werden dem Verbrennungsofen 10 mit dem dazugehörenden Kamin 11 zugeführt, und dort unter Zusatz von Verbrennungsluft 12 verbrannt. Im Verbrennungsofen 10 ist vorteilhaft ein Wäremtauscher 13 zur Wärmerückgewinnung angeordnet. Das Verbrennungsabgas aus dem Verbrennungsofen wird als Strom 14 teilweise dem Abgasstrom 15 zugemischt. Dieser Abgasstrom tritt nach der Mischung mit der gemäß der Erfindung erhöhten Temperaturen auf 40° bis 150 °C in die Atmosphäre aus.

## Patentansprüche

1. Verfahren zur Erhöhung der Temperatur des Abgases aus der Abgaswaschanlage bei der Herstellung von Phthalsäureanhydrid durch katalytische Luftoxidation von Naphthalin oder o-Xylol, dadurch gekennzeichnet, daß dem Abgas vor dem Austritt in die Atmosphäre Verbrennungsabgas aus dem Verbrennungsofen für Rückstände aus den PSA- und Maleinsäureanhydrid-Destillationseinheiten zugemischt wird.

2. Verfahren nach Anspruch 1, dadurch gekennzeichnet, daß dem Abgas nur ein Teil des Verbrennungsabgases zugemischt wird.

3. Verfahren nach Anspruch 1, dadurch gekennzeichnet, daß dem Abgas das gesamte Verbrennungsabgas zugemischt wird.

## Claims

1. A process for raising the temperature of the waste gas from the waste gas scrubbing stage of the production of phthalic anhydride by catalytic air oxidation of naphthalene or o-xylene, which comprises admixing the waste gas before exit into the open with incineration waste gas from the incineration furnace for residues from the phthalic anhydride and maleic anhydride distillation units.

2. A process as claimed in claim 1, wherein the waste gas is admixed with only part of the incineration waste gas.

3. A process as claimed in claim 1, wherein the waste gas is admixed with all the incineration waste gas.

## Revendications

1. Procédé pour élever la température des gaz d'échappement des installations de lavage des gaz d'échappement pour la fabrication d'anhydride phtalique par oxydation catalytique à l'air de naphtalène ou de o-xylène, caractérisé par le fait qu'on mélange aux gaz d'échappement, avant la sortie dans l'atmosphère, des gaz d'échappement de combustion provenant du four de combustion pour résidus provenant des unités de distillation d'anhydride phtalique et maléique.

2. Procédé selon la revendication 1, caractérisé par le fait qu'on ne mélange aux gaz d'échappement qu'une partie des gaz d'échappement de combustion.

3. Procédé selon la revendication 1, caractérisé par le fait qu'on mélange aux gaz d'échappement la totalité des gaz d'échappement de combustion.